# EUROPEAN PATENT SPECIFICATION

(11) **EP 1 396 233 B1**
(45) Date of publication and mention of the grant of the patent: **26.03.2014**
(21) Application number: 03255564.1
(22) Date of filing: 05.09.2003
(51) Int. Cl.: A61B 19/00, A61B 5/04, A61N 1/36

(54) **Positioning system for neurological procedures in the brain**
Positionierungssystem für neurologische Eingriffe im Gehirn
Système de positionnement pour les interventions neurologiques dans le cerveau

(30) Priority: 06.09.2002 US 236437
(43) Date of publication of application: 10.03.2004
(73) Proprietor: Biosense Webster, Inc., Diamond Bar, CA 91765 (US)
(72) Inventor: Yaron, Uri, Zichron-Yaacov, 30900 (IL)
(74) Representative: Small, Gary James

(56) References cited:
- EP-A- 1 306 050
- WO-A-02/19243
- WO-A-98/33451
- WO-A-98/39669
- WO-A1-96/05768
- US-A- 6 061 587
- US-A- 6 165 164
- US-B1- 6 240 308
- US-B1- 6 285 898

## Description

The present invention relates generally to intrabody tracking systems, and specifically to devices for tracking the position and orientation of a medical instrument in the brain.

Many surgical, diagnostic, therapeutic and prophylactic medical procedures require the precise placement of objects such as sensors, treatment units, tubes, catheters, implants and other devices within the body on a temporary or permanent basis. In particular, advances have been made in the field of neuroscience by development of new techniques, many of which require the use of implantable devices or other invasive procedures for treatment of a variety of abnormal conditions associated with the neurological activities and morphology of the brain. These developments include:
- Deep Brain Stimulation (DBS) therapy, which is delivered by an implanted medical device, similar to a cardiac pacemaker, which uses mild electrical stimulation to modify brain signals that cause unwanted effects. Targeted cells are stimulated in the subthalamic nucleus (STN) via electrodes that are surgically implanted in the brain and connected to a neurostimulator implanted elsewhere in the body.
- Thalamotomy, in which a lesion is made in the thalamus (an area of the brain that produces tremors). Thalamotomy has been shown to effectively reduce tremors in some patients.
- Pallidotomy, which is a surgical operation that destroys the pallidum. The purpose of this procedure is to relieve involuntary movements or muscular rigidity as, for example, in Parkinson's disease.
- Fetal neural implant (or nigral implant), which is an experimental technique that involves transplanting fetal tissue into the brain to replace degenerated nerves.

It has been demonstrated that Deep Brain Stimulation (DBS) at high frequencies (100 Hz or higher) can alleviate, diminish, or completely terminate symptoms of tremor, rigidity, akinesia (loss or impairment of voluntary activity) or hemiballism (violent uncontrollable movements of one side of the body). US-A-5,716,377 and US-A-5,833,709 describe techniques for stimulating the brain to treat movement disorders that result in abnormal motor behavior. A sensor is used to detect the symptoms resulting from the motion disorder and an algorithm analyzes the output from the sensor in order to regulate the stimulation delivered to the brain.

US-A-5,713,923 and US-A-5,978,702 describe techniques using drugs and electrical stimulation to treat neurological disorders, including epilepsy, by means of an implantable signal generator and electrode coupled to an implantable pump and catheter. A sensor is used to detect a seizure or symptoms resulting from the onset of a seizure. A microprocessor analyzes the output from the sensor in order to regulate the stimulation and drug dosage delivered to the neural tissue.

US-A-5,800,474 discloses a method for preventing seizures experienced by persons with epilepsy. High frequency electrical stimulation pulses are supplied to the STN via electrodes that are surgically implanted in the brain and connected to a neurostimulator implanted elsewhere in the body.

US-A-5,975,085, which is referred to herein as the '085 patent, describes techniques for using drugs and/or electrical stimulation for treating schizophrenia by means of an implantable signal generator and electrode and an implantable pump and catheter. The catheter is surgically implanted in the brain to infuse the drugs, and one or more electrodes are surgically implanted in the brain to provide electrical stimulation.

US-A-6,109,269 describes techniques using drugs, electrical stimulation or both, in a manner analogous to that of the '085 patent, in order to treat addictions. US-A-6,128,537 describes techniques similar to those of the '085 patent for treating anxiety disorder.

US-A-5,735,814 and US-A-5,814,014 describe techniques for infusing drugs into the brain to treat neurodegenerative disorders using an implantable pump and catheter. The drugs are capable of altering the level of excitation of the neurons in the brain. A sensor is used to detect an attribute of the nervous system which reflects the hyperexcitation of the nerve cell projecting onto the degenerating nerve cells. A microprocessor algorithm analyzes the output from the sensor in order to regulate the amount of drug delivered to the brain.

The use of brain implants and other invasive procedures for diagnostic and therapeutic treatments requires a high level of precision in order to reduce damage to surrounding tissue and deleterious side effects. US-A-5,843,148 describes a stimulation lead which includes a high spatial resolution tip carrying a plurality of electrodes that can be used in stimulating small neurological brain targets. US-A-5,865,843 describes a neurological lead for transmission of therapeutic drugs and/or electrical signals to body organs such as the spinal column or brain. More specifically, this patent describes the mechanisms and methods by which such leads are secured to the human body.

US-A- 6,314,310 describes apparatus for determining the position of a surgical tool during X-ray guided surgery.

US-A- 6,076,008 describes a system for determining the position of a probe relative to the head of a patient during surgery, and displaying corresponding scan images of the same position in the head.

US-A-6,246,898 and US-A-5,797,849 describe a method for carrying out medical procedures, including in the brain, using a 3-D tracking and imaging system.

US-A-6,298,262 describes a method for positioning a surgical instrument during stereotactic surgery using a guidance fixture and a remote sensing device such as a camera.

US-A-5,517,990 describes the use of a stereotaxic wand in conjunction with a guide to designate a location and trajectory at which a surgical tool is applied to a patient. During use of the system, the location and trajectory of the wand are superimposed on a diagnostic image on a monitor.

US-A- 6,226,547 describes a catheter tracking system for locating and tracking a position of a catheter within a body using reference transducers.

The following references may be useful:
Hutchison WD et al., "Neurophysiological identification of the subthalamic nucleus in surgery for Parkinson's disease," Ann Neurol, 44, 622-628 (1988)
Gross RE et al., "Advances in neurostimulation for movement disorders," Neurol Res, 22, 247-258 (2000)
Montgomery EB et al., "Mechanisms of deep brain stimulation and future technical developments," Neurol Res, 22, 259-66 (2000)
Benabid AL et al., "Future prospects of brain stimulation," Neurol Res, 22, 237-246 (2000)
Kupsch A et al., "Neurological interventions in the treatment of idiopathic Parkinson disease: Neurostimulation and neural implantation," J Mol Med, 77, 178-184 (1999)
Alesch F et al., "Stimulation of the ventral intermediate thalamic nucleus in tremor dominated Parkinson's disease and essential tremor," Acta Neurochi (wien), 136, 75-81 (1995)

WO 98/33451 discloses a system with a multimodality instrument for tissue identification that includes a computer-controlled motor driven heuristic proble with a multisensory tip including a microelectrode to measure brain electrical activity or an irrigator, and wherein the instrument includes a location sensor.

### SUMMARY OF THE INVENTION

It is an object of some aspects of the present invention to provide improved apparatus for real-time determination of the location and orientation of a medical instrument within the brain during a medical procedure.

It is a further object of some aspects of the present invention to provide improved apparatus for accurately positioning a medical instrument at a target site within the brain during a medical procedure.

It is yet a further object of some aspects of the present invention to provide apparatus for medical instrument positioning within the brain that can be integrated with existing commercially-available mapping support systems.

It is still a further object of some aspects of the present invention to provide apparatus to enable simultaneous access to electrophysiological and anatomical data of the brain.

It is yet an additional object of some aspects of the present invention to provide apparatus to enable more effective and safe treatment of neurological disorders.

In preferred embodiments of the present invention, apparatus for performing a medical procedure in a patient's brain comprises a medical instrument, such as a probe, catheter, needle, or pacemaker lead, which comprises a plurality of location sensors and one or more electrodes for sensing electrical activity in the brain. Preferably, the instrument also comprises a therapeutic or diagnostic element affixed thereto. Using images typically acquired prior to the procedure, the instrument is inserted into the brain in the vicinity of tissue of interest. Using a combination of absolute location information, anatomical location information, and electrical activity information, the instrument is guided precisely to the location of the target tissue, and the procedure is performed using the therapeutic or diagnostic element.

Typically, target regions within the brain at which procedures are performed are on the order of a few millimeters in size. A combination of both electrophysiological and anatomical data is preferred in these embodiments to accurately identify a target region and its borders within the brain. Anatomical information alone is generally insufficient, because the borders between different electrophysiological regions are, in many cases, not definable by standard imaging tools such as CT or MRI. The addition of measured electrical activity in the target region enables the accurate identification of the target tissue. It is therefore particularly advantageous that these applications of the present invention are able to provide real-time feedback of the location of the probe and the electrical activity at that location in order to determine the position of the probe with respect to local electrophysiological activity at that position. That is, data obtained using techniques that indicate particular x-y-z probe coordinates, even when overlaid on a CT image, are not necessarily sufficient to indicate that the probe is in contact with desired tissue. Similarly, data obtained using techniques that indicate local electrophysiological activity without x-y-z probe coordinates are not able to provide easy guidance to the target region, especially when the probe is mounted on a flexible catheter. However, the combination of these coordinates with electrophysiological data, as provided by these embodiments of the present invention, provides the physician with a high level of confidence that the probe is moving towards and eventually is in contact with the desired target.

Real-time analysis of the signals received from the location sensors and electrodes on the probe within the brain allows the creation of an electrical map indicating the different physiological regions of the brain. Overlaying this electrical map on a CT-generated anatomical map enables precise location and orientation of the probe and allows the surgeon to guide the element to the desired therapeutic or diagnostic site. This is a significant advantage over prior art techniques which have no means of continually updating both the location of the tip of the probe and electrical activity at the location of the probe with respect to the CT images. It is noted that whereas some preferred embodiments of the present invention are described herein with respect to the use of CT images, the application of the described technologies in combination with other imaging modalities (e.g., MRI) is also considered to be within the scope of the present invention.

Synchronization of instrument location information with images showing the environment surrounding the instrument are preferably performed using methods and apparatus known in the art, such as those described in the above-cited patents to Ben-Haim, Bucholz, and Vesely et al. In a preferred embodiment, a stereotactic frame is fixed to the patient's head and location measurements are made with respect to this frame prior to and during the procedure. Typically, a set of CT images is acquired prior to surgery in order to determine the location of the target region at which the procedure is to be performed. Preferably, features in the image are registered with coordinates ofthe location sensing system in order to enable synchronization. Typically, a reference position on the frame, possibly including a transducer, is used as a feature of one or more images in order to aid in performing the registration process.

To determine the absolute location of the instrument and assist in placing it at the desired site, methods and apparatus are preferably but not necessarily utilized which are described in EP-A-1,321,097 and EP-A-1,325,708. Preferably, one or more external electromagnetic or ultrasound transducers are placed at fixed positions with respect to the stereotactic frame. The transducers are driven by a control unit to transmit energy towards, or to receive energy transmitted by, the sensors on the instrument, in order to facilitate calculation of the location and orientation, with respect to the frame, of the instrument and the element attached thereto that performs the diagnostic or therapeutic function. Alternatively or additionally, methods and apparatus known in the art are used to facilitate location sensing.

In some applications of the present invention, the element performing the diagnostic or therapeutic function may be adapted for long term implantation within the brain, while for other applications, the element is removed at the end of the procedure.

There is therefore provided, in accordance with the present invention, apparatus for use in a brain of a subject, wherein the apparatus is defined in claim 1 herein.

Preferably, the instrument is adapted to be guided to a target location in the brain responsive to the electrophysiological information and the determined position of the instrument.

For some applications, the control unit is adapted to create an electrical map indicating at least two physiological regions of the brain, responsive to the electrical activity signal and the location signal.

In a preferred embodiment, the location sensor is adapted to transmit the location signal by wireless communication.

At least one of the electrodes is adapted to be coupled to a distal tip of the instrument. The location sensor is adapted to be coupled at a distal tip of the instrument.

For some applications, the instrument is adapted to facilitate a fetal neural implant, responsive to the control unit determining the electrophysiological information regarding the tissue at the position.

In a preferred embodiment, the control unit is adapted to determine the position of the instrument with respect to an image of the brain acquired prior to insertion of the instrument into the brain. Alternatively or additionally, the control unit is adapted to determine the position of the instrument with respect to an image of the brain acquired while the instrument is in the brain.

For some applications, the image of the brain includes a CT scan, and the control unit is adapted to determine the position of the instrument with respect to the CT scan. For other applications, the image of the brain includes an MRI image, and the control unit is adapted to determine the position of the instrument with respect to the MRI image.

Preferably, the control unit is adapted to register one or more identifiable anatomical features in the image, and to correlate the position of the instrument with the image responsive to the registration.

The instrument includes a delivery element, adapted to deliver a pharmaceutical at a target location responsive to the electrical signal and the location signal.

Typically, the location sensor includes an electromagnetic transducer. In this case, the apparatus preferably includes one or more external electromagnetic radiators, adapted to be located at respective positions external to the subject and to transmit energy towards the location sensor. Alternatively, the location sensor includes an ultrasound transducer, and the apparatus includes one or more external ultrasound transducers, adapted to be located at respective positions external to the subject, and to transmit ultrasound energy towards the location sensor.

In a preferred embodiment, the apparatus includes a diagnostic element coupled to the instrument.

For some applications, the instrument includes a catheter. In accordance with a preferred embodiment of the present invention, the catheter includes a vascular catheter, adapted to be guided responsive to the location signal to a target location in the brain, through cerebral vasculature of the subject. For example, the catheter may be adapted to be guided responsive to the location signal to a target location in the brain through a venous circulation of the brain and subsequently through tissue of the brain.

The apparatus typically includes a stereotactic frame which is adapted to be fixed to a head of the subject, and the control unit determines the position of the instrument with respect to the frame.

Preferably, the apparatus includes a current-driving electrode, adapted to be placed by the instrument at a target location of the brain and to apply a therapeutic current to the target location. In a preferred embodiment, the control unit is adapted to drive the current-driving electrode to apply the therapeutic current. In a preferred application, the current-driving electrode is adapted to apply Deep Brain Stimulation therapy to the target location. Alternatively or additionally, the current-driving electrode is adapted to apply current configured for treatment of a motor disorder or a mental disorder. Further alternatively or additionally, the current-driving electrode is adapted to apply current configured for performing ablation at the target location, e.g., so as to facilitate performing thalamotomy or performing pallidotomy.

In some preferred embodiments, the current-driving electrode is adapted for long-term implantation in the brain.

There is further described, although not claimed as part of the present invention, a method for performing a medical procedure in a brain of a subject, including:
inserting an instrument into the brain;
sensing electrical activity of the brain in a vicinity of an electrical-activity sensing site on the instrument, and transmitting an electrical activity signal responsive thereto; sensing, at a location-sensing site on the instrument, a location of the instrument, and transmitting a location signal responsive thereto;
determining, responsive to the location signal, a position of the instrument with respect to an image of the brain; and
determining, responsive to the electrical activity signal and the determination of the position, electrophysiological information regarding tissue at the position.

The present invention will be more fully understood from the following detailed description of the preferred embodiments thereof, taken together with the drawing, in which:

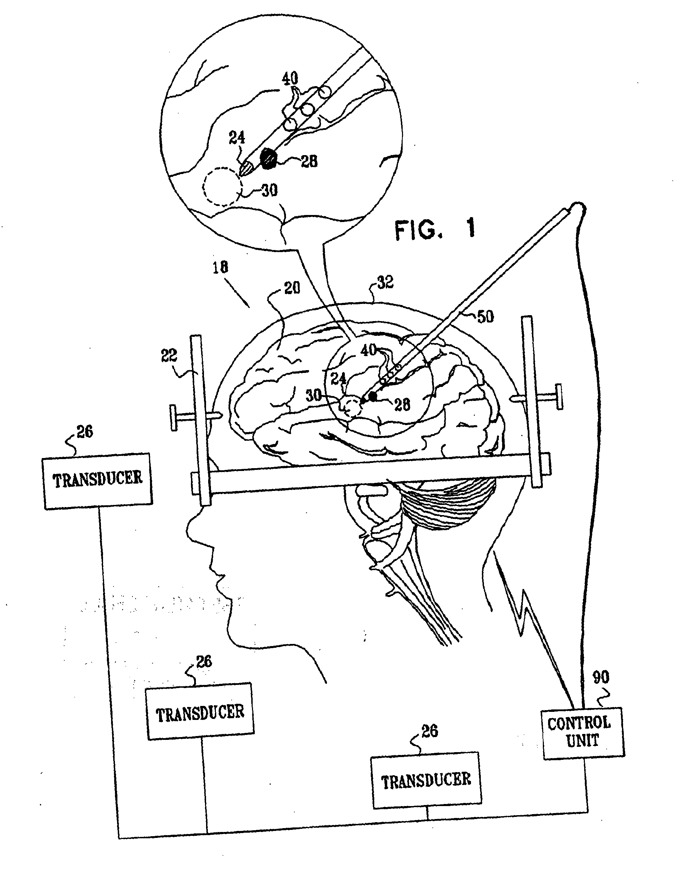

Fig. 1 is a schematic, pictorial illustration of a system for tracking the electrophysiological position of a medical instrument in the brain, in accordance with a preferred embodiment of the present invention.

Although the invention is described below in connection with a method for performing a medical procedure, no claim is made herein to any such method.

Fig. 1 is a schematic, pictorial illustration of a system 18 for tracking the position and orientation of an instrument 50, such as a probe, catheter, needle, pharmaceutical-delivery element or pacemaker lead, in a brain 20 of a subject, in accordance with a preferred embodiment of the present invention. Instrument 50 comprises one or more location sensors 40 preferably located at or near the distal end of instrument 50 for determining position and orientation coordinates of the distal end of instrument 50 and one or more electrodes 28 on instrument 50 for sensing electrical activity of tissue, such as brain tissue, and performing anatomical and/or viability mapping. Preferably, the one or more location sensors include at least one or more electromagnetic inductive coils responsive to electromagnetic fields generated by transducers such as electromagnetic field generators 26 in accordance with description below. For purposes of this disclosure, the term "transducers 26" means either electromagnetic field generators and/or electromagnetic field receivers or alternatively, ultrasound transmitters and/or receivers. Preferably, instrument 50 also comprises a therapeutic or diagnostic element 24 affixed thereto for providing therapy and/or a diagnostic procedure on target tissue of interest 30. Instrument 50 is inserted into brain 20 in the vicinity of target tissue of interest 30. Using a combination of absolute location information (derived from position and orientation coordinates) generated by use of location sensors 40 and electrical activity information generated by use of electrodes 28, instrument 50 is guided to the precise position of target tissue 30, for instance, as determined by electrophysiological data recorded thereat or a desired location coordinate which can be a predetermined position as identified on an image of brain 20 or target tissue 30. Typically, a therapeutic or diagnostic procedure is performed on the target tissue using element 24. This procedure may be, for example, a procedure described in any of the references cited in the Background section of the present patent application.

Synchronization of absolute location information of instrument 50 with images showing the environment surrounding instrument 50 is preferably performed using methods and apparatus known in the art, such as those described in the above-cited patents to Ben-Haim, Bucholz, and Vesely et al. In a preferred embodiment, a stereotactic frame 22 is fixed to the patient's head 32 and location measurements are made with respect to frame 22 prior to and during the procedure. Typically, a set of CT, MRI, SPECT, ultrasound or other imaging modality images are acquired prior to surgery in order to determine the location of the region of target tissue 30 within the brain at which the procedure is to be performed. Preferably, features in the image are registered with position and orientation coordinates of the location sensing system in order to enable synchronization. Typically, a reference position on frame 22, or a reference position sensor on frame 22, possibly including a transducer (not shown), is used as a feature of one or more images in order to aid in performing the registration process. For some applications, the reference position sensor comprises an electromagnetic position sensor having one or more inductive coils.

To determine the absolute location of instrument 50 and assist in placing it at the desired site, i.e., target tissue 30, methods and apparatus are preferably utilized which are described in the above-cited EP-A-1,321,097 and EP-A-1,325,708. Preferably, the one or more external electromagnetic field generators 26 (or alternatively, ultrasound transducers when location sensor 40 is one or more ultrasonic transducers) are placed at fixed positions external to the patient's body with respect to stereotactic frame 22, and location sensors 40 are preferably located on the distal end of instrument 50. Transducers 26 are driven by a control unit 90, preferably at a plurality of frequencies, to transmit energy towards location sensors 40 on instrument 50, by, in the electromagnetic field embodiment, generating electromagnetic fields, or in the ultrasound embodiment, transmitting ultrasonic waves, or, in other embodiments, generating appropriate energy fields. Alternatively, transducers 26 receive energy transmitted by location sensors 40. Responsive to the received energy, control unit 90 calculates the location, i.e., position and orientation coordinates, of location sensors 40, distal end of instrument 50, and element 24 attached thereto, with respect to frame 22. Alternatively or additionally, methods and apparatus known in the art are used to facilitate location sensing. According to some of these methods, location sensors 40 are located on the proximal end of instrument 50. Alternatively or additionally, transducers 26 receive energy transmitted by location sensors 40 on instrument 50, i.e. transducers 26 serve as electromagnetic receivers for electromagnetic fields generated by location sensors 40, in the electromagnetic embodiment, and as ultrasound receivers for ultrasonic waves transmitted by location sensors 40, in the ultrasound embodiment.

Although for some applications instrument 50 is generally rigid, as shown in the figure and as is common in the prior art, for other applications, the instrument is generally flexible, e.g., by being made of a flexible material. In some applications, the instrument comprises a vascular catheter, which is preferably guided to target tissue 30 through the cerebral vasculature using the overlay of location data on the image (for example the CT or MRI image), and instrument 50 is subsequently verified to be at target tissue 30 by the electrophysiological data provided to the control unit 90 by the one or more electrodes 28. Advantageously, the techniques described herein permit the use of such a flexible instrument 50 without requiring it to be mounted to stereotactic frame 22, and, therefore, without the need to pass instrument 50 through a substantial amount of intermediate brain tissue of brain 20 while approaching target tissue 30. For some procedures, instrument 50 is passed through the venous circulation of brain 20 to a site close to target tissue 30, and then passed out of the venous circulation to target tissue 30, typically without passing through a significant amount of brain tissue following exit from the venous circulation. If local bleeding is anticipated responsive to this last step, then techniques of bleeding control known in the art are preferably used, e.g., pharmaceutical agents, electrocautery or mechanical elements to temporarily or permanently block the site where the instrument exited the venous circulation.

In some applications of the present invention such as, for example, chronic deep brain stimulation, element 24 comprises a stimulator or another element, which may be adapted for long term implantation in brain 20, while for other applications such as, for example, biopsy, element 24 is removed from the brain at the end of the procedure.

Typically, target regions 30 within brain 20 at which procedures are performed are on the order of a few millimeters in size. Thus, the position and orientation coordinate signals and information (position and orientation coordinates) generated by the one or more location sensors 40 are extremely useful for this purpose. A combination of both electrophysiological and anatomical data is preferred in these embodiments to accurately identify the target region 30 and its borders within the brain 20. Anatomical information alone is generally insufficient, because the borders between different electrophysiological regions are, in many cases, not definable by standard imaging tools such as CT or MRI. The addition of measured electrical activity in the target region enables the accurate identification of the target tissue. It is also particularly advantageous that the system 18 used for these applications in accordance with these embodiments of the present invention is able to provide real-time feedback of the location (including position and orientation coordinates) of the instrument or probe 50 and the electrical activity at that location (provided by the one or more electrodes 28) in order to determine the position of the probe with respect to local electrophysiological activity at that position. That is, data obtained using techniques that indicate particular x-y-z position coordinates and orientation coordinates, such as pitch, yaw and roll, even when overlaid on a CT or MRI image, for example, may not be necessarily sufficient to indicate that the probe is indeed in contact with desired tissue. Similarly, data obtained using techniques that indicate local electrophysiological activity without x-y-z position and pitch, yaw and/or roll orientation coordinates may not be able to provide easy guidance to target region 30, especially when instrument 50 is a flexible catheter. However, the combination of these position and orientation coordinates with electrophysiological data (provided by one or more electrodes 28), as provided by these embodiments of the present invention, provides the physician with a high level of confidence that instrument 50 is moving towards and eventually is in contact with the desired target 30.

Real-time analysis of the signals received by control unit 90 from the one or more location sensors 40 and one or more electrodes 28 on instrument 50 within brain 20 allows the creation of an anatomical and/or electrophysiological map, such as an electrical map, indicating the different physiological regions of brain 20, and including target tissue 30. Overlaying this electrical map on the image, such as the CT- or MRI-generated image (anatomical image map) enables precise location, i.e., position and orientation of the distal end of instrument 50, and allows the surgeon to guide element 24 to the desired therapeutic or diagnostic site, i.e., target tissue 30. This is a significant advantage over prior art techniques which have no means of continually updating both the location of the distal end of an instrument and the electrical activity at the location of the instrument with respect to the tissue and CT images. It is noted that whereas some preferred embodiments of the present invention are described herein with respect to the use of CT images, the application of the described technologies in combination with other imaging modalities (e.g., MRI) is also considered to be within the scope of the present invention.

Synchronization of instrument location information with images showing the environment surrounding instrument 50 are preferably performed using methods and apparatus known in the art, such as those described in the above-cited patents to Ben-Haim, Bucholz, and Vesely et al. In a preferred embodiment, stereotactic frame 22 is fixed to the patient's head, and location measurements are made with respect to this frame 22 prior to and during the procedure. Typically, a set of images, such as CT images, is acquired prior to surgery in order to determine the location of target region 30 at which the procedure is to be performed. Preferably, features in the image are registered with coordinates of location sensing system 18, in order to enable synchronization. Typically, a reference position or reference position sensor, such as described above, is provided on the frame 22, and is used as a feature of one or more images in order to aid in performing the registration process.

To determine the absolute location of instrument 50 and assist in placing it at the desired site or target tissue 30, methods and apparatus are preferably but not necessarily utilized which are described in EP-A-1,321,097 and EP-A-1,325,708. Preferably, these include the use of the one or more external electromagnetic field or ultrasound transducers (generators) 26, placed at fixed positions with respect to stereotactic frame 22. The transducers 26 are driven by control unit 90, as described above, to transmit energy towards, or to receive energy transmitted by, the sensors on the instrument, depending on the embodiment, in order to facilitate calculation of the location, i.e., position and orientation coordinates, with respect to the frame, of instrument 50 and element 24 attached thereto that performs the diagnostic or therapeutic function. In some applications of the present invention, element 24 performing the diagnostic or therapeutic function may be adapted for long term implantation within the brain, while for other applications, element 24 is removed at the end of the procedure. In a preferred embodiment, the one or more location sensors 40 are adapted to be both powered and/or able to transmit the location signal to the control unit 90 by wireless communication, so that system 18 serves as a telemetric system.

At least one of electrodes 28 is adapted to be coupled to the distal tip of instrument 50 through connection techniques such as those known in the art. Location sensor 40 or one or more location sensors 40 is adapted to be coupled or connected at the distal tip of the instrument 50, also through techniques known in the art.

For some applications, instrument 50 is used to facilitate a fetal neural implant, in conjunction with control unit 90 using both electrophysiological information (from the one or more electrodes 28) and location information (position and orientation coordinates) regarding target tissue 30 at or near the site targeted for implantation of the fetal tissue.

Additionally, for some applications, control unit 90 is adapted to register one or more identifiable anatomical features of the tissue, for example the tissue of brain 20, in the image, and to correlate the location (position and orientation coordinates) of the instrument 50 with the image responsive to and in alignment with the registration.

In a preferred embodiment, instrument 50 uses element 24 as a delivery element, such as an injection needle or infusion port, adapted to deliver a pharmaceutical or therapeutical agent, including a therapeutical peptide, protein, nucleic acid or other biological molecular compound at target site 30 based on and responsive to the electrical signals (provided by the one or more electrodes 28) and the location signal (provided by the one or more location sensors 40).

Typically, location sensor 40 includes an electromagnetic transducer which uses one or more inductive coils. In this case, the apparatus preferably includes one or more external electromagnetic radiators, adapted to be located at respective positions external to the subject and to transmit energy in the form of a generated different, respective electromagnetic field towards location sensor 40. Alternatively, location sensor 40 includes an ultrasound transducer, and system 18 includes one or more external ultrasound transducers 26, adapted to be located at respective positions external to the patient or subject, and to transmit ultrasound energy (in the form of ultrasonic waves) towards the location sensor 40.

In a preferred embodiment of the present invention, system 18 includes a current-driving electrode (not shown), adapted to be placed by instrument 50 at target location 30 of brain 20 in order to apply a therapeutic current to the target location 30. In a preferred embodiment, control unit 90 operatively communicates with the current driving electrode, and is adapted to drive the current-driving electrode to apply the therapeutic current. In a preferred application, the current-driving electrode is adapted to apply "deep brain stimulation" therapy to target location 30. Alternatively or additionally, the current-driving electrode is adapted to apply current configured for treatment of a motor disorder or a mental disorder. Further alternatively or additionally, the current-driving electrode is adapted to apply current configured for performing ablation at the target location, e.g., so as to facilitate performing thalamotomy or performing pallidotomy.

In some preferred embodiments, the current-driving electrode is adapted for long-term implantation in the brain.

It will be understood by one skilled in the art that these embodiments of the present invention can be applied in the treatment of a variety of neurological and other disorders associated with the morphology and activity of the brain, including, but not limited to, those described hereinabove.

## Claims

1. Apparatus (18) for use in a brain (20) of a subject, comprising:
an instrument (50) adapted to be inserted into the brain (20);
one or more electrodes (28) on the instrument (50) at the distal end of the instrument (50) for sensing electrical activity of the brain (20) and transmitting an electrical activity signal responsive thereto;
one or more location sensors (40) located at the distal end of the instrument (50) for transmitting a location signal indicative of a location of the instrument (50); and
a control unit (90) for analyzing the electrical activity signal and the location signal and determining, responsive to the analysis, a position of the instrument (50) with respect to an image of the brain, and electrophysiological information regarding tissue (30) at the position;
wherein the instrument (50) includes a delivery element (24) for delivering a pharmaceutical at a target location (30) responsive to the electrical signal and the location signal.

2. Apparatus according to claim 1, wherein the instrument (50) is adapted to be guided to a target location (30) in the brain (20) responsive to the electrophysiological information and the determined position of the instrument (50).

3. Apparatus according to claim 1 or claim 2, wherein the control unit (90) is adapted to create an electrical map indicating at least two physiological regions of the brain, responsive to the electrical activity signal and the location signal.

4. Apparatus according to any one of claims 1 to 3, wherein the one or more location sensors (40) is adapted to transmit the location signal by wireless communication.

5. Apparatus according to any one of claims 1 to 4, wherein the instrument (50) is adapted to facilitate a fetal neural implant, responsive to the control unit (90) determining the electrophysiological information regarding the tissue (30) at the position.

6. Apparatus according to any one of claims 1 to 5, wherein the control unit (90) is adapted to determine the position of the instrument (50) with respect to an image of the brain (20) acquired prior to insertion of the instrument (50) into the brain (20).

7. Apparatus according to any one of claims 1 to 6, wherein the control unit (90) is adapted to determine the position of the instrument (50) with respect to an image of the brain (20) acquired while the instrument (50) is in the brain (20).

8. Apparatus according to any one of claims 1 to 7, wherein the image of the brain (20) includes a CT scan, and wherein the control unit (90) is adapted to determine the position of the instrument (50) with respect to the CT scan.

9. Apparatus according to any one of claims 1 to 8, wherein the image of the brain includes an MRI image, and wherein the control unit (90) is adapted to determine the position of the instrument with respect to the MRI image.

10. Apparatus according to any one of claims 1 to 9, wherein the control unit (90) is adapted to register one or more identifiable anatomical features in the image, and to correlate the position of the instrument (50) with the image responsive to the registration.

11. Apparatus according to any one of claims 1 to 10, wherein the one or more location sensors (40) comprises an electromagnetic transducer.

12. Apparatus according to any one of claims 1 to 10, wherein the one or more location sensors (40) comprises an ultrasound transducer.

13. Apparatus according to claim 11 or claim 12, comprising one or more external transducers, adapted to be located at respective positions external to the subject, and to transmit energy towards the one or more location sensors (40).

14. Apparatus according to any one of claims 1 to 13, comprising a diagnostic element (24) coupled to the instrument (50).

15. Apparatus according to any one of claims 1 to 14, wherein the instrument (50) comprises a catheter.

16. Apparatus according to claim 15, wherein the catheter comprises a vascular catheter, adapted to be guided responsive to the location signal to a target location (30) in the brain (20), through cerebral vasculature of the subject.

17. Apparatus according to claim 15 or claim 16, wherein the catheter is adapted to be guided responsive to the location signal to a target location (30) in the brain (20) through a venous circulation of the brain and subsequently through tissue of the brain (20).

18. Apparatus according to any one of claims 1 to 17, comprising a stereotactic frame (22) which is adapted to be fixed to a head (32) of the subject, wherein the control unit (90) determines the position of the instrument (50) with respect to the frame (22).

19. Apparatus according to any one of claims 1 to 18, comprising a current-driving electrode, adapted to be placed by the instrument (50) at a target location (30) of the brain (20) and to apply a therapeutic current to the target location (30).

20. Apparatus according to claim 19, wherein the control unit (90) is adapted to drive the current-driving electrode to apply the therapeutic current.

21. Apparatus according to claim 19 or claim 20, wherein the current-driving electrode is adapted to apply Deep Brain Stimulation therapy to the target location (30).

22. Apparatus according to claim 19 or claim 20, wherein the current-driving electrode is adapted to apply current configured for treatment of a motor disorder.

23. Apparatus according to claim 19 or claim 20, wherein the current-driving electrode is adapted to apply current configured for treatment of a mental disorder.

24. Apparatus according to claim 19 or claim 20, wherein the current-driving electrode is adapted to apply current configured for performing ablation at the target location (30).

25. Apparatus according to claim 24, wherein the current-driving electrode is adapted to apply current configured for performing thalamotomy at the target location (30).

26. Apparatus according to claim 24, wherein the current-driving electrode is adapted to apply current configured for performing pallidotomy at the target location (30).

27. Apparatus according to claim 19 or claim 20, wherein the current-driving electrode is adapted for long-term implantation in the brain (20).

## Patentansprüche

1. Gerät (18) zur Verwendung in einem Gehirn (20) eines Patienten, umfassend:
ein Instrument (50) zur Einführung in das Gehirn (20);
eine oder mehrere Elektroden (28) auf dem Instrument (50) am distalen Ende des Instruments (50) zur Messung der elektrischen Aktivität des Gehirns (20) und
Übertragung eines elektrischen Aktivitätssignals als Reaktion darauf;
einen oder mehrere Positionssensoren (40), die am distalen Ende des Instruments (50) angeordnet sind, zur Übertragung eines Positionssignals, das einen Hinweis auf eine Position des Instruments (50) liefert; und
eine Steuereinheit (90) zur Auswertung des elektrischen Aktivitätssignals und des Positionssignals und zur Ermittlung, als Reaktion auf die Auswertung, einer Position des Instruments (50) bezüglich eines Bildes des Gehirns, und von elektrophysiologischen Informationen bezüglich von Gewebe (30) an der Position;
worin das Instrument (50) ein Abgabeelement (24) zur Abgabe eines pharmazeutischen Mittels an einer Zielstelle (30) als Reaktion auf das elektrische Signal und das Positionssignal aufweist.

2. Gerät nach Anspruch 1, worin das Instrument (50) als Reaktion auf die elektrophysiologische Information und die ermittelte Position des Instruments (50) zu einer Zielstelle (30) im Gehirn (20) geführt werden kann.

3. Gerät nach Anspruch 1 oder Anspruch 2, worin die Steuereinheit (90) als Reaktion auf das elektrische Aktivitätssignal und das Positionssignal eine elektrische Karte erzeugen kann, die auf mindestens zwei physiologische Regionen im Gehirn hinweist.

4. Gerät nach einem der Ansprüche 1 bis 3, worin der eine oder die mehreren Positionssensoren (40) das Positionssignal durch drahtlose Kommunikation übertragen kann.

5. Gerät nach einem der Ansprüche 1 bis 4, worin das Instrument (50) als Reaktion auf die Ermittlung der elektrophysiologischen Information bezüglich des Gewebes (30) an der Position durch die Steuereinheit (90) ein fötales neuronales Implantat erleichtern kann.

6. Gerät nach einem der Ansprüche 1 bis 5, worin die Steuereinheit (90) die Position des Instruments (50) bezüglich eines Bildes des Gehirns (20), das vor Einführung des Instruments (50) in das Gehirn (20) erfasst wurde, ermitteln kann.

7. Gerät nach einem der Ansprüche 1 bis 6, worin die Steuereinheit (90) die Position des Instruments (50) bezüglich eines Bildes des Gehirns (20), das erfasst wurde, während sich das Instrument (50) im Gehirn (20) befindet, ermitteln kann.

8. Gerät nach einem der Ansprüche 1 bis 7, worin das Bild des Gehirns (20) eine CT-Aufnahme aufweist und worin die Steuereinheit (90) die Position des Instruments (50) bezüglich der CT-Aufnahme ermitteln kann.

9. Gerät nach einem der Ansprüche 1 bis 8, worin das Bild des Gehirns eine MRT-Aufnahme aufweist und worin die Steuereinheit (90) die Position des Instruments bezüglich der MRT-Aufnahme ermitteln kann.

10. Gerät nach einem der Ansprüche 1 bis 9, worin die Steuereinheit (90) eines oder mehrere identifizierbare anatomische Merkmale in dem Bild registrieren kann und die Position des Instruments (50) als Reaktion auf die Registrierung mit dem Bild korrelieren kann.

11. Gerät nach einem der Ansprüche 1 bis 10, worin der eine oder die mehreren Positionssensoren (40) einen elektromagnetischen Wandler umfasst bzw. umfassen.

12. Gerät nach einem der Ansprüche 1 bis 10, worin der eine oder die mehreren Positionssensoren (40) einen Ultraschallwandler umfasst bzw. umfassen.

13. Gerät nach Anspruch 11 oder Anspruch 12, umfassend einen oder mehrere externe Wandler, die an jeweiligen Positionen außerhalb des Patienten angeordnet werden können und die Energie zu dem einen oder zu den mehreren Positionssensoren (40) übertragen können.

14. Gerät nach einem der Ansprüche 1 bis 13, ein mit dem Instrument (50) verbundenes Diagnoseelement (24) umfassend.

15. Gerät nach einem der Ansprüche 1 bis 14, worin das Instrument (50) einen Katheter umfasst.

16. Gerät nach Anspruch 15, worin der Katheter einen Gefäßkatheter umfasst, der als Reaktion auf das Positionssignal zu einer Zielstelle (30) im Gehirn (20) durch die Zerebralgefäße des Patienten geführt werden kann.

17. Gerät nach Anspruch 15 oder Anspruch 16, worin der Katheter als Reaktion auf das Positionssignal zu einer Zielstelle (30) im Gehirn (20) durch einen venösen Kreislauf des Gehirns und anschließend durch Gewebe des Gehirns (20) geführt werden kann.

18. Gerät nach einem der Ansprüche 1 bis 17, einen Stereotaxierahmen (22) umfassend, der an einem Kopf (32) des Patienten befestigt werden kann, worin die Steuereinheit (90) die Position des Instruments (50) bezüglich des Rahmens (22) ermittelt.

19. Gerät nach einem der Ansprüche 1 bis 18, eine stromtreibende Elektrode umfassend, die von dem Instrument (50) an einer Zielstelle (30) des Gehirns (20) platziert werden kann und einen therapeutischen Strom auf die Zielstelle (30) aufbringen kann.

20. Gerät nach Anspruch 19, worin die Steuereinheit (90) die stromtreibende Elektrode zum Aufbringen des therapeutischen Stroms antreiben kann.

21. Gerät nach Anspruch 19 oder Anspruch 20, worin die stromtreibende Elektrode eine tiefe Gehirnstimulationstherapie auf die Zielstelle (30) aufbringen kann.

22. Gerät nach Anspruch 19 oder Anspruch 20, worin die stromtreibende Elektrode Strom zur Behandlung einer motorischen Störung aufbringen kann.

23. Gerät nach Anspruch 19 oder Anspruch 20, worin die stromtreibende Elektrode Strom zur Behandlung einer geistigen Störung aufbringen kann.

24. Gerät nach Anspruch 19 oder Anspruch 20, worin die stromtreibende Elektrode Strom zur Durchführung einer Ablation an der Zielstelle (30) aufbringen kann.

25. Gerät nach Anspruch 24, worin die stromtreibende Elektrode Strom zur Durchführung einer Thalamotomie an der Zielstelle (30) aufbringen kann.

26. Gerät nach Anspruch 24, worin die stromtreibende Elektrode Strom zur Durchführung einer Pallidotomie an der Zielstelle (30) aufbringen kann.

27. Gerät nach Anspruch 19 oder Anspruch 20, worin die stromtreibende Elektrode Strom zur Langzeitimplantation im Gehirn (20) ausgelegt ist.

## Revendications

1. Appareil (18) destiné à une utilisation dans le cerveau (20) d'un patient, comprenant :
un instrument (50) conçu pour être inséré dans le cerveau (20) ;
une ou plusieurs électrodes (28) situées sur l'instrument (50) au niveau de l'extrémité distale de l'instrument (50) pour capter une activité électrique du cerveau (20) et transmettre un signal d'activité électrique en réponse à celle-ci ;
un ou plusieurs capteurs de position (40) situés au niveau de l'extrémité distale de l'instrument (50) pour transmettre un signal de position indicatif d'une position de l'instrument (50) ; et
une unité de contrôle (90) pour analyser le signal d'activité électrique et le signal de position et déterminer, en réponse à l'analyse, une position de l'instrument (50) relativement à une image du cerveau, et des informations électrophysiologiques concernant les tissus (30) au niveau de la position ;
où l'instrument (50) comprend un élément de délivrance (24) pour délivrer un produit pharmaceutique au niveau d'une position cible (30) en réponse au signal électrique et au signal de position.

2. Appareil selon la revendication 1, dans lequel l'instrument (50) est conçu pour être guidé vers une position cible (30) dans le cerveau (20) en réponse aux informations électrophysiologiques et à la position déterminée de l'instrument (50).

3. Appareil selon la revendication 1 ou la revendication 2, dans lequel l'unité de contrôle (90) est conçue pour créer une carte électrique indiquant au moins deux régions physiologiques du cerveau, en réponse au signal d'activité électrique et au signal de position.

4. Appareil selon l'une quelconque des revendications 1 à 3, dans lequel le ou les capteurs de position (40) sont conçus pour transmettre le signal de position par une communication sans fil.

5. Appareil selon l'une quelconque des revendications 1 à 4, dans lequel l'instrument (50) est conçu pour faciliter un implant neural foetal, en réponse à l'unité de contrôle (90) déterminant les informations électrophysiologiques concernant les tissus (30) au niveau de la position.

6. Appareil selon l'une quelconque des revendications 1 à 5, dans lequel l'unité de contrôle (90) est conçue pour déterminer la position de l'instrument (50) relativement à une image du cerveau (20) acquise antérieurement à l'insertion de l'instrument (50) dans le cerveau (20).

7. Appareil selon l'une quelconque des revendications 1 à 6, dans lequel l'unité de contrôle (90) est conçue pour déterminer la position de l'instrument (50) relativement à une image du cerveau (20) acquise pendant que l'instrument (50) est dans le cerveau (20).

8. Appareil selon l'une quelconque des revendications 1 à 7, dans lequel l'image du cerveau (20) comprend un tomodensitogramme, et où l'unité de contrôle (90) est conçue pour déterminer la position de l'instrument (50) relativement au tomodensitogramme.

9. Appareil selon l'une quelconque des revendications 1 à 8, dans lequel l'image du cerveau comprend une image d'IRM, et où l'unité de contrôle (90) est conçue pour déterminer la position de l'instrument relativement à l'image d'IRM.

10. Appareil selon l'une quelconque des revendications 1 à 9, dans lequel l'unité de contrôle (90) est conçue pour enregistrer une ou plusieurs caractéristiques anatomiques identifiables dans l'image, et pour corréler la position de l'instrument (50) à l'image en réponse à l'enregistrement.

11. Appareil selon l'une quelconque des revendications 1 à 10, dans lequel le ou les capteurs de position (40) comprennent un transducteur électromagnétique.

12. Appareil selon l'une quelconque des revendications 1 à 10, dans lequel le ou les capteurs de position (40) comprennent un transducteur à ultrasons.

13. Appareil selon la revendication 11 ou la revendication 12, comprenant un ou plusieurs transducteurs externes, conçus pour être situés à des positions respectives externes du patient, et pour transmettre une énergie vers le ou les capteurs de position (40).

14. Appareil selon l'une quelconque des revendications 1 à 13, comprenant un élément de diagnostique (24) couplé à l'instrument (50).

15. Appareil selon l'une quelconque des revendications 1 à 14, dans lequel l'instrument (50) comprend un cathéter.

16. Appareil selon la revendication 15, dans lequel le cathéter comprend un cathéter vasculaire, conçu pour être guidé en réponse au signal de position vers une position cible (30) dans le cerveau (20), à travers le système vasculaire cérébral du patient.

17. Appareil selon la revendication 15 ou la revendication 16, dans lequel le cathéter est conçu pour être guidé en réponse au signal de position vers une position cible (30) dans le cerveau (20) à travers une circulation veineuse du cerveau et subséquemment à travers les tissus du cerveau (20).

18. Appareil selon l'une quelconque des revendications 1 à 17, comprenant un cadre stéréotaxique (22) qui est conçu pour être fixé à la tête (32) du patient, où l'unité de contrôle (90) détermine la position de l'instrument (50) par rapport au cadre (22).

19. Appareil selon l'une quelconque des revendications 1 à 18, comprenant une électrode conductrice de courant, conçue pour être placée par l'instrument (50) au niveau d'une position cible (30) du cerveau (20) et pour appliquer un courant thérapeutique au niveau de la position cible (30).

20. Appareil selon la revendication 19, dans lequel l'unité de contrôle (90) est conçue pour commander l'électrode conductrice de courant pour appliquer le courant thérapeutique.

21. Appareil selon la revendication 19 ou la revendication 20, dans lequel l'électrode conductrice de courant est conçue pour appliquer une thérapie de stimulation cérébrale profonde au niveau de la position cible (30).

22. Appareil selon la revendication 19 ou la revendication 20, dans lequel l'électrode conductrice de courant est conçue pour appliquer un courant configuré pour un traitement d'un trouble moteur.

23. Appareil selon la revendication 19 ou la revendication 20, dans lequel l'électrode conductrice de courant est conçue pour appliquer un courant configuré pour un traitement d'un trouble mental.

24. Appareil selon la revendication 19 ou la revendication 20, dans lequel l'électrode conductrice de courant est conçue pour appliquer un courant configuré pour exécuter une ablation au niveau de la position cible (30).

25. Appareil selon la revendication 24, dans lequel l'électrode conductrice de courant est conçue pour appliquer un courant configuré pour exécuter une thalamotomie au niveau de la position cible (30).

26. Appareil selon la revendication 24, dans lequel l'électrode conductrice de courant est conçue pour appliquer un courant configuré pour exécuter une pallidectomie au niveau de la position cible (30).

27. Appareil selon la revendication 19 ou la revendication 20, dans lequel l'électrode conductrice de courant est conçue pour une implantation à long terme dans le cerveau (20).
